# EUROPEAN PATENT SPECIFICATION

(11) **EP 1 267 847 B1**
(45) Date of publication and mention of the grant of the patent: **04.02.2004**
(21) Application number: 00978627.8
(22) Date of filing: 14.11.2000
(51) Int. Cl.: A61K 31/416, A61P 27/06

(54) **5HT 2 AGONISTS FOR CONTROLLING IOP AND TREATING GLAUCOMA**
5-HT2 AGONISTEN ALS MITTEL ZUR KONTROLLE DES AUGENINNENDRUCKS UND ZUR BEHANDLUNG VON GLAUKOM
AGONISTES DE 5HT 2 PERMETTANT DE CONTROLER LA PRESSION INTRA-OCULAIRE ELEVEE ET DE TRAITER LE GLAUCOME

(30) Priority: 17.03.2000 US 190288 P
(43) Date of publication of application: 02.01.2003
(73) Proprietor: Alcon Inc., 6331 Hünenberg (CH); YAMANOUCHI PHARMACEUTICAL CO., LTD., Tokyo 103 (JP)
(72) Inventor: MAY, Jesse, A., Fort Worth, TX 76109 (US); DANTANARAYANA, Anura, P., Fort Worth, TX 76132 (US)
(74) Representative: Best, Michael, Dr.
(86) International application number: PCT/US2000/031246
(87) International publication number: WO 2001/070207

(56) References cited:
- WO-A-01/40183
- WO-A-98/30548
- OSBORNE N N: "SEROTONIN AND MELATONIN IN THE IRIS/CILIARY PROCESSES AND THEIR INVOLVEMENT IN INTRAOCULAR PRESSURE" ACTA NEUROBIOLOGIAE EXPERIMENTALIS, WARSZAW, PL, vol. 54, no. SUPPL, 1994, pages 57-64, XP000878634 ISSN: 0065-1400

## Description

The present invention is directed to the use of 1 -(2-aminopropyl)-indazol-6-ol for lowering and controlling intraocular pressure (IOP) and treating glaucoma.

### Background of the Invention

The disease state referred to as glaucoma is characterized by a permanent loss of visual function due to irreversible damage to the optic nerve. The several morphologically or functionally distinct types of glaucoma are typically characterized by elevated IOP, which is considered to be causally related to the pathological course of the disease. Ocular hypertension is a condition wherein intraocular pressure is elevated but no apparent loss of visual function has occurred; such patients are considered to be a high risk for the eventual development of the visual loss associated with glaucoma. Some patients with glaucomatous field loss have relatively low intraocular pressures. These so called normotension or low tension glaucoma patients can also benefit from agents that lower and control IOP. If glaucoma or ocular hypertension is detected early and treated promptly with medications that effectively reduce elevated intraocular pressure, loss of visual function or its progressive deterioration can generally be ameliorated. Drug therapies that have proven to be effective for the reduction of intraocular pressure include both agents that decrease aqueous humor production and agents that increase the outflow facility. Such therapies are in general administered by one of two possible routes, topically (direct application to the eye) or orally.

There are some individuals who do not respond well when treated with certain existing glaucoma therapies. There is, therefore, a need for other topical therapeutic agents that control IOP.

The compound, 1-(2-aminopropyl)-indazol-6-ol, is disclosed in WO98/30548. Example 46 within the application discloses the S-enantiomer of 1-(2-aminopropyl)-indazol-6-ol. The utility cited in the application is for treating central nervous system diseases, such as, sexual disorders, genital insufficiency, appetite regulation disorders, anxiety, depression, and sleep disorders. No ophthalmic indications are disclosed.

### Summary of the Invention

The present invention is directed to compositions of 1-(2-aminopropyl)-indazol-6-ol and its use for lowering and controlling IOP and treating glaucoma.

### Description of Preferred Embodiments

Surprisingly, it has been found that 1-(2-aminopropyl)-indazol-6-ol ("Compound") and its S-(+)-isomer, when dosed at 300 µg, in the lasered monkey model of ocular hypertension causes a significant decrease in IOP as shown in the table set forth below. Intraocular pressure was determined with an Alcon Pneumatonometer after light comeal anesthesia with 0.1% proparacaine. Eyes were washed with saline after each measurement. After a baseline IOP measurement, test compound was instilled in one 30 µL aliquot to the right eyes only of nine cynomolgus monkeys. Vehicle was instilled in the right eyes of six additional animals. Subsequent IOP measurements were taken at 1, 3, and 6 hours. A compound is considered efficacious in this model of ocular hypertension if there is a decrease in the baseline IOP of the lasered eye (O.D.) of at least 20% following topical administration.

| **IOP Response to 1-(2-Aminopropyl)-indazol-6-ol and its S(+)-isomer** | | | | | |
|---|---|---|---|---|---|
| | **Dose (µg)** | **Baseline IOP (mmHg)** | **IOP Response % Change (ΔmmHg)** | | |
| | | | **1 hr** | **3 hr** | **6 hr** |
| Compound | 300 | 40.1 | -17.6(7.3) | -28.1 (11.8) | -33.8 (14.6) |
| | vehicle | 40.5 | -6.5 (3.0) | -13.6 (5.7) | -8.1 (3.8) |
| S-(+)-isomer | 300 | 40.3 | -15.0 (6.4) | -35.3 (14.8) | -40.8(17.1) |
| | vehicle | 38.0 | -3.1 (1.8) | -6.8 (3.3) | -4.7 (2.8) |

The S-isomer of 1-(2-aminopropyl)-indazol-6-ol is the preferred isomer for lowering and controlling IOP and treating glaucoma.

The Compound can be incorporated into various types of ophthalmic formulations for delivery to the eye (e.g., topically, intracamerally, or via an implant). It is preferably incorporated into topical ophthalmic formulations for delivery to the eye. The Compound may be combined with ophthalmologically acceptable preservatives, surfactants, viscosity enhancers, penetration enhancers, buffers, sodium chloride, and water to form an aqueous, sterile ophthalmic suspension or solution. Ophthalmic solution formulations may be prepared by dissolving the Compound in a physiologically acceptable isotonic aqueous buffer. Further, the ophthalmic solution may include an ophthalmologically acceptable surfactant to assist in dissolving the Compound. Furthermore, the ophthalmic solution may contain an agent to increase viscosity, such as, hydroxymethylcellulose, hydroxyethylcellulose, hydroxypropylmethylcellulose, methylcellulose, polyvinylpyrrolidone, or the like, to improve the retention of the formulation in the conjunctival sac. Gelling agents can also be used, including, but not limited to, gellan and xanthan gum. In order to prepare sterile ophthalmic ointment formulations, the Compound is combined with a preservative in an appropriate vehicle, such as, mineral oil, liquid lanolin, or white petrolatum. Sterile ophthalmic gel formulations may be prepared by suspending the active ingredient in a hydrophilic base prepared from the combination of, for example, carbopol-974, or the like, according to the published formulations for analogous ophthalmic preparations; preservatives and tonicity agents can be incorporated.

The Compound is preferably formulated as a topical ophthalmic suspension or solution, with a pH of about 5 to 8. The Compound will normally be contained in these formulations in an amount 0.01% to 5% by weight, but preferably in an amount of 0.1% to 2% by weight. Thus, for topical presentation 1 to 2 drops of these formulations would be delivered to the surface of the eye 1 to 4 times per day according to the discretion of a skilled clinician.

The compounds can also be used in combination with other agents for treating glaucoma, such as, but not limited to, β-blockers (e.g., timolol, betaxolol, levobetaxolol, carteolol, levobunolol, propranolol), carbonic anhydrase inhibitors (e.g., brinzolamide and dorzolamide), α1 antagonists (e.g. nipradolol), α2 agonists (e.g., iopidine and brimonidine), miotics (e.g., pilocarpine and epinephrine), prostaglandin analogs (e.g., latanoprost, travoprost, unoprostone, and compounds set forth in U.S. Patent Nos. 5,889,052; 5,296,504; 5,422,368; and 5,151,444, "hypotensive lipids" (e.g., bimatoprost and compounds set forth in 5,352,708), and neuroprotectants (e.g., compounds from U.S. Patent No. 4,690,931, particularly eliprodil and R-eliprodil, as set forth in a pending application U.S.S.N. 60/203,350 (WO 01/85152), and appropriate compounds from WO94/13275, including memantine.

The following topical ophthalmic formulations are useful according to the present invention administered 1-4 times per day according to the discretion of a skilled clinician.

### EXAMPLE 1

| **Ingredients** | **Amount (wt %)** |
|---|---|
| 1-(2-Aminopropyl)-indazol-6-ol (S-(+)-isomer) | 0.01 - 2% |
| Hydroxypropyl methylcellulose | 0.5% |
| Dibasic sodium phosphate (anhydrous) | 0.2% |
| Sodium chloride | 0.5% |
| Disodium EDTA (Edetate disodium) | 0.01% |
| Polysorbate 80 | 0.05% |
| Benzalkonium chloride | 0.01% |
| Sodium hydroxide / Hydrochloric acid | For adjusting pH to 7.3 - 7.4 |
| Purified water | q.s. to 100% |

### EXAMPLE 2

| **Ingredients** | **Amount (wt %)** |
|---|---|
| 1-(2-Aminopropyl)-indazol-6-ol | 0.01 - 2% |
| Methyl cellulose | 4.0% |
| Dibasic sodium phosphate (anhydrous) | 0.2% |
| Sodium chloride | 0.5% |
| Disodium EDTA (Edetate disodium) | 0.01% |
| Polysorbate 80 | 0.05% |
| Benzalkonium chloride | 0.01 % |
| Sodium hydroxide / Hydrochloric acid | For adjusting pH to 7.3 - 7.4 |
| Purified water | q.s. to 100% |

### EXAMPLE 3

| **Ingredients** | **Amount (wt %)** |
|---|---|
| 1-(2-Aminopropyl)-indazol-6-ol (S-(+)-isomer) | 0.01 - 2% |
| Guar gum | 0.4- 6.0% |
| Dibasic sodium phosphate (anhydrous) | 0.2% |
| Sodium chloride | 0.5% |
| Disodium EDTA (Edetate disodium) | 0.01% |
| Polysorbate 80 | 0.05% |
| Benzalkonium chloride | 0.01 % |
| Sodium hydroxide / Hydrochloric acid | For adjusting pH to 7.3 - 7.4 |
| Purified water | q.s. to 100% |

### EXAMPLE 4

| **Ingredients** | **Amount (wt %)** |
|---|---|
| 1-(2-Aminopropyl)-indazol-6-ol | 0.01 - 2% |
| White petrolatum and mineral oil and lanolin | Ointment consistency |
| Dibasic sodium phosphate (anhydrous) | 0.2% |
| Sodium chloride | 0.5% |
| Disodium EDTA (Edetate disodium) | 0.01 % |
| Polysorbate 80 | 0.05% |
| Benzalkonium chloride | 0.01% |
| Sodium hydroxide / Hydrochloric acid | For adjusting pH to 7.3 - 7.4 |

## Claims

1. Use of 1-(2-aminopropyl)-indazol-6-ol for the preparation of a medicament for controlling intraocular pressure.

2. Use of the S-(+)-isomer of 1-(2-aminopropyl)-indazol-6-ol for the preparation of a medicament for controlling intraocular pressure.

3. Use of a compound according to claim 1 or 2 in combination with one or more other agents for treating glaucoma, selected from β-blockers, carbonic anhydrase inhibitors, α₁ antagonists, α₂ agonists, miotics, prostaglandin analogs, hypotensive lipids, and neuroprotectants.

4. Use of a compound according to claim 1 or 2 in combination with one or more of the following: timolol, betaxolol, levobetaxolol, carteolol, levobunolol, propranolol, brinzolamide, dorzolamide, nipradolol, iopidine, brimonidine, pilocarpine, epinephrine, latanoprost, travoprost, unoprostone, bimatoprost, eliprodil and R-eliprodil.

5. A topical ocular composition for controlling intraocular pressure comprising a pharmaceutically effective amount of 1-(2-aminopropyl)-indazol-6-ol and a pharmaceutically acceptable carrier or diluent.

6. The composition of claim 5 comprising the S-(+)-isomer of 1-(2-aminopropyl)-indazol-6-ol.

7. The composition of claim 5 wherein the concentration of 1-(2-aminopropyl)-indazol-6-ol is from 0.01 to 5 weight percent.

8. The composition of claim 7 wherein the concentration is from 0.1 to 2 weight percent.

9. The composition of claim 6 wherein the concentration of S-(+)-isomer of 1-(2-aminopropyl)-indazol-6-ol is from 0.01 to 5 weight percent.

10. The composition of claim 9 wherein the concentration is from 0.1 to 2 weight percent.

11. The composition according to one of claims 5-10 containing additionally one or more other agents for treating glaucoma.

12. The composition according to claim 11 in which the other agent is selected from β-blockers, carbonic anhydrase inhibitors, α₁ antagonists, α₂ agonists, miotics, prostaglandin analogs, hypotensive lipids, and neuroprotectants.

13. The composition according to claim 12 in which the other agent is one or more of the following: timolol, betaxolol, levobetaxolol, carteolol, levobunolol, propranolol, brinzolamide, dorzolamide, nipradolol, iopidine, brimonidine, pilocarpine, epinephrine, latanoprost, travoprost, unoprostone, bimatoprost, eliprodil and R-eliprodil.

## Patentansprüche

1. Verwendung von 1-(2-Aminopropyl)indazol-6-ol zur Herstellung eines Arzneimittels zur Kontrolle des Intraokulardrucks.

2. Verwendung des S-(+)-Isomers von 1-(2-Aminopropyl)indazol-6-ol zur Herstellung eines Arzneimittels zur Kontrolle des Intraokulardrucks.

3. Verwendung einer Verbindung nach Anspruch 1 oder Anspruch 2 in Kombination mit einem oder mehreren anderen Mitteln zur Behandlung von Glaukom ausgewählt aus β-Blockern, Carboanhydrasehemmern, α1-Antagonisten, α2-Agonisten, Miotika, Prostaglandinanaloga, blutdrucksenkenden Lipiden und neuroprotektiven Mitteln.

4. Verwendung einer Verbindung nach Anspruch 1 oder Anspruch 2 in Kombination mit einem oder mehreren der folgenden Mittel: Timolol, Betaxolol, Levobetaxolol, Carteolol, Levobunolol, Propranolol, Brinzolamid, Dorzolamid, Nipradolol, Iopidin, Brimonidin, Pilocarpin, Epinephrin, Latanoprost, Travoprost, Unoproston, Bimatoprost, Eliprodil und R-Eliprodil.

5. Topische Okularzusammensetzung zur Kontrolle des Intraokulardrucks enthaltend eine pharmazeutisch wirksame Menge von 1-(2-Aminopropyl)indazol-6-ol und einen pharmazeutisch annehmbaren Träger oder ein Verdünnungsmittel.

6. Zusammensetzung nach Anspruch 5, enthaltend das S-(+)-Isomer von 1-(2-Aminopropyl)indazol-6-ol.

7. Zusammensetzung nach Anspruch 5, wobei die Konzentration von 1-(2-Aminopropyl)indazol-6-ol 0,01 bis 5 Gew.-% ist.

8. Zusammensetzung nach Anspruch 7, wobei die Konzentration 0,1 bis 2 Gew.-% ist.

9. Zusammensetzung nach Anspruch 6, wobei die Konzentration des S-(+)-Isomers von 1-(2-Aminopropyl)indazol-6-ol 0,01 bis 5 Gew.-% ist.

10. Zusammensetzung nach Anspruch 9, wobei die Konzentration 0,1 bis 2 Gew.-% ist.

11. Zusammensetzung nach einem der Ansprüche 5 bis 10 enthaltend zusätzlich ein oder mehrere Mittel zur Behandlung von Glaukom.

12. Zusammensetzung nach Anspruch 11, wobei das andere Mittel ausgewählt ist aus β-Blockern, Carboanhydrasehemmern, α1-Antagonisten, 0.2-Agonisten, Miotika, Prostaglandinanaloga, blutdrucksenkenden Lipiden und neuroprotektiven Mitteln.

13. Zusammensetzung nach Anspruch 12, wobei das andere Mittel eines oder mehrere der folgenden ist: Timolol, Betaxolol, Levobetaxolol, Carteolol, Levobunolol, Propranolol, Brinzolamid, Dorzolamid, Nipradolol, Iopidin, Brimonidin, Pilocarpin, Epinephrin, Latanoprost, Travoprost, Unoproston, Bimatoprost, Eliprodil und R-Eliprodil.

## Revendications

1. Utilisation de 1-(2-aminopropyl)-indazol-6-ol pour la préparation d'un médicament pour le contrôle de la pression intraoculaire.

2. Utilisation du S-(+)-isomère du 1-(2-aminopropyl)-indazol-6-ol pour la préparation d'un médicament pour le contrôle de la pression intraoculaire.

3. Utilisation d'un composé selon la revendication 1 ou 2 en combinaison avec un ou plusieurs autres agents pour le traitement du glaucome, choisis parmi des β-bloquants, des inhibiteurs d'anhydrase carbonique, des antagonistes α1, des agonistes α2, des myotiques, des analogues de la prostaglandine, des lipides hypotenseurs et des neuroprotecteurs.

4. Utilisation d'un composé selon la revendication 1 ou 2 en combinaison avec un ou plusieurs des composés suivants: timolol, betaxolol, lévobetaxolol, cartéolol, lévobunolol, propanolol, brinzolamide, dorzolamide, nipradolol, iopidine, brimonidine, pilocarpine, épinéphrine, lanatoprost, travoprost, unoprostone, bimatoprost; éliprodil et R-éliprodil.

5. Composition oculaire topique pour le contrôle de la pression intraoculaire, comprenant une quantité pharmaceutiquement efficace de 1-(2-aminopropyl)-indazol-6-ol et un excipient ou un diluant pharmaceutiquement acceptable.

6. Composition selon la revendication 5, comprenant le S-(+)-isomère du 1-(2-aminopropyl)-indazol-6-ol.

7. Composition selon la revendication 5, dans laquelle la concentration de 1-(2-aminopropyl)-indazol-6-ol est de 0,01 à 5% en poids.

8. Composition selon la revendication 7, dans laquelle la concentration est de 0,1 à 2% en poids.

9. Composition selon la revendication 6, dans laquelle la concentration du (S-(+)-isomère du 1-(2-aminopropyl)-indazol-6-ol est de 0,01 à 5% en poids.

10. Composition selon la revendication 9, dans laquelle la concentration est de 0,1 à 2% en poids.

11. Composition selon l'une quelconque des revendications 5 à 10, contenant en outre un ou plusieurs autres agents pour le traitement du glaucome.

12. Composition selon la revendication 11, dans laquelle l'autre agent est choisi parmi des β-bloquants, des inhibiteurs d'anhydrase carbonique, des antagonistes α1, des agonistes α2, des myotiques, des analogues de la prostaglandine, des lipides hypotenseurs, et des neuroprotecteurs.

13. Composition selon la revendication 12, dans laquelle l'autre agent est l'un ou plusieurs des composés suivants: timolol, betaxolol, lévobetaxolol, cartéolol, lévobunolol, propanolol, brinzolamide, dorzolamide, nipradolol, iopidine, brimonidine, pilocarpine, épinéphrine, lanatoprost, travoprost, unoprostone, bimatoprost; éliprodil et R-éliprodil.
